# EUROPÄISCHE PATENTANMELDUNG

(11) **EP 1 801 585 A1**
(43) Veröffentlichungstag der Anmeldung: **27.06.2007**
(21) Anmeldenummer: 06126118.6
(22) Anmeldetag: 14.12.2006
(51) Int. Cl.: G01N 33/487

(54) **Behälter für medizinisches Verbrauchsmaterial, mit Trockenmittelvorrat**

(30) Priorität: 23.12.2005 EP 05028293
(71) Anmelder: F. Hoffmann-la Roche AG, 4070 Basle (CH); Roche Diagnostics GmbH, 68305 Mannheim (DE)
(72) Erfinder: Thoes, Bruno, 66286, Quierschied (DE); Manser, Udo, 68723, Schwetzingen (DE); Leiniger, Helmut, 68259, Mannheim (DE)
(74) Vertreter: Dick, Alexander

(57) **Zusammenfassung**

Die Erfindung bezieht sich auf ein Umverpackungssystem für medizinisches Verbrauchsmaterial, insbesondere Testelemente (58). Das Umverpackungssystem umfasst eine Umverpackung (10), die einen ersten Trockenmittelvorrat (18) aufnimmt, und ein Magazin (22) mit einem unverbrauchten Testelementvorrat (30). Im geschlossenen Zustand (24) der Umverpackung (10) wirkt der erste Trockenmittelvorrat (18) der Umverpackung (10) über eine eine Wasserdampfdiffusion ermöglichende Verbindung (96) auf das Innere des Magazins (22) ein.

## Beschreibung

Die Erfindung bezieht sich auf ein Umverpackungssystem für medizinisches Verbrauchsmaterial, insbesondere Testelemente, die in einem aus der Umverpackung entnehmbaren Magazin aufgenommen sind.

### Stand der Technik

DE 198 19 407 A1 bezieht sich auf einen Teststreifenbehälter für Messgeräte, die mit Einwegteststreifen arbeiten. Diese Einwegteststreifen werden in der Regel einem Sensor zur Messung zugeführt. Der Behälter, in dem die Einwegteststreifen aufgenommen sind, umfasst zwei Teile, in dessen erstem Teil die Teststreifen magaziniert sind und in dessen zweitem Teil die verbrauchten Teststreifen gesammelt werden.

In einem Magazin aufgenommenes medizinisches Verbrauchsmaterial, wie z. B. Testelemente, die zur Blutzuckermessung zur Laktatmessung oder auch zur Cholesterinbestimmung in Messgeräten verwendet werden, wird in einer z. B. als Umhüllung ausgebildeten Verpackung verpackt, um das medizinische Verbrauchsmaterial vor Umgebungseinflüssen zu schützen. Testelemente für medizinische Zwecke reagieren besonders empfindlich auf Temperaturschwankungen und insbesondere auf Feuchtigkeit. Daher sind Magazine, in welchen im Allgemeinen eine Anzahl von Testelementen aufgenommen ist, in die Umverpackung eingeschweißt, um einen Feuchtigkeitseintritt in die Umverpackung und damit eine Schädigung des in der Umverpackung aufgenommenen Magazins mit den Testelementen gegen eindringende Feuchtigkeit zu verhindern.

Die in der Regel wasserdampfdicht ausgeführte Umverpackung, die zu Verkaufszwecken in einer Verkaufsverpackung aufgenommen ist, enthält einen Trockenmittelvorrat. Auch das Magazin, in welchem die Testelemente aufgenommen sind, enthält einen Trockenmittelvorrat. Der Trockenmittelvorrat im Magazin mit den Testelementen dient dazu, während der Zeitspanne zwischen der Herstellung der Testelemente bis zu deren Verbrauch durch den Anwender die im Magazin enthaltenen Testelemente vor Wasserdampfbeladung zu schützen. Damit kann erreicht werden, dass die Testelemente eine von der Lagerungsdauer unabhängige Haltbarkeit aufweisen, da während dieser Zeitspanne der Trockenmittelvorrat innerhalb des die Testelemente aufnehmenden Magazins einen diffundierenden Wasserdampf aufnimmt und von den im Magazin enthaltenen Testelementen fernhält.

Ist das Magazin z. B. kassettenförmig ausgebildet und enthält Wickelspulen für auf einem Transportband aufgenommene Einzeltestelemente, so wird in der Regel in dem Teil, in dem sich die unverbrauchten, in Form einer gewickelten Spule befindenden Testelemente aufgenommen sind, eine Trockenmittelmenge eingebracht. Die Kassette wird nach der Bestückung mit dem Transportband, auf welches die Einzeltestelemente aufgebracht sind, versiegelt und in eine z. B. als Aluminiumtopf ausgebildete Umverpackung eingelegt und zusammen mit einem ebenfalls in die Umverpackung eingebrachten Trockenmittel in der Umverpackung eingeschweißt. Bei geschlossener Umverpackung stellt der darin enthaltene Trockenmittelvorrat während der Lagerung sicher, dass keine Umgebungsfeuchte während der Lagerung auf die Kassette einwirkt und in diese eindringt.

Von Nachteil bei der oben dargestellten Lösung gemäß des derzeitigen Standes der Technik ist der Umstand, dass beim Herstellungsprozess sowohl das Trockenmittel als auch die Testelemente unvermeidlich mit Wasserdampf belastet werden. Nach Fertigstellung des Magazins, dies bedeutet nach Beladung des Magazins mit einer Anzahl von Testelementen, sind die darin aufgenommenen Testelemente und das im Magazin enthaltene Trockenmittel mit herstellungsbedingter Feuchtigkeit teilbeladen. Dies bedeutet, dass der im Magazin enthaltene Trockenmittelvorrat nur noch eine Restaufnahmekapazität für eindringende Feuchtigkeit hat. Der im Magazin enthaltene Trockenmittelvorrat trocknet zuerst das Innere des Magazins, die noch verbleibende Restaufnahmekapazität des im Magazin enthaltenen Trockenmittelvorrates dient zur Sicherstellung der Haltbarkeit der Testelemente im Magazin.

### Darstellung der Erfindung

Der Erfindung liegt die Aufgabe zugrunde, ein Umverpackungssystem für medizinisches Verbrauchsmaterial, wie z. B. Testelemente, bereitzustellen, welches die Haltbarkeit (On-Board-Stability) der Testelemente erhöht.

Der erfindungsgemäß vorgeschlagenen Lösung folgend, kann während der Lagerung des die Testelemente aufnehmenden Magazins in der Umverpackung eine Verbindung zwischen dem Trockenmittelvorrat in der Umverpackung und dem Trockenmittelvorrat im die Testelemente aufnehmenden Magazin geschaffen werden, welche, solange die Umverpackung geschlossen ist, eine Verbindung zwischen dem in der Umverpackung aufgenommenen Trockenmittel und dem im Magazin enthaltenen Trockenmittel herstellt. Die Verbindung zwischen dem Trockenmittelvorrat der Umverpackung und dem im Magazin enthaltenen Trockenmittelvorrat kann z. B. durch einen Spacer oder Abstandhalter geschaffen werden, der einerseits mit der Umverpackung verbunden ist, und welcher andererseits zwischen einer Dichtung und dem Gehäuse des in der Umverpackung aufgenommenen Magazins mit den Testelementen liegt.

Durch den Spacer oder Abstandhalter, der z. B. als ein Band mit einem porösen Abschnitt als eine Gewebestruktur, als ein Vlies oder ein Fadengeflecht ausgeführt sein kann, wird eine Öffnung zwischen der Dichtung des Magazins und dem Innenraum der Umverpackung geschaffen, die so lange existiert, solange das die Testelemente aufnehmende Magazin in der wasserdampfdichten Umhüllung der Umverpackung aufgenommen ist.

Solange das Magazin mit dem darin aufgenommenen Trockenmittelvorrat in der Umverpackung verbleibt, kann über die mittels des Spacers beziehungsweise Abstandhalters erweiterte Öffnung der in der Umverpackung aufgenommene Trockenmittelvorrat Feuchtigkeit aus dem Magazin heraus mit aufnehmen. In der Regel ist der in der Umverpackung aufgenommene Feuchtmittelvorrat wesentlich größer als der Feuchtmittelvorrat, der im die Testelemente enthaltenden Magazin aufgenommen ist. Durch die erfindungsgemäß vorgeschlagene Lösung kann der in der Umverpackung aufgenommene Trockenmittelvorrat dazu herangezogen werden, einerseits die herstellungsbedingte Feuchtigkeitsbeladung der im Magazin enthaltenen Testelemente herabzusetzen und lagerungszeitbedingt eindiffundierende Feuchtigkeit in die Umverpackung zu absorbieren.

In einem bevorzugten Ausführungsbeispiel wird, sobald die Umverpackung geöffnet wird, die zuvor durch den Spacer oder Abstandhalter zwischen dem Gehäuse des Magazins und der Dichtung und dem in der Umverpackung enthaltenen Trockenmittelvorrat geschaffene Öffnung auf eine fertigungstechnisch bedingte Restgröße verschlossen. In diesem bevorzugten Ausführungsbeispiel der vorliegenden Erfindung wird beim Öffnen der Umverpackung der mit der Umverpackung verbundene Spacer beziehungsweise Abstandhalter aus der Öffnung zwischen der Dichtung und dem Magazin herausgezogen, wodurch die Öffnung ihre Restgröße annimmt. Wird der Spacer beziehungsweise Abstandhalter aus einem porösen, eine Wasserdampfdiffusion ermöglichenden Material gefertigt, so vermag der in der Umverpackung aufgenommene Trockenmittelvorrat Wasserdampf aus dem Inneren des Magazins zu ziehen, der über den zwischen dem Gehäuse des Magazins mit der Dichtung aufgenommenen Spacer beziehungsweise Abstandhalter im Hohlraum der Umverpackung durch den dort enthaltenen größeren Trockenmittelvorrat absorbiert werden kann. In einem weiteren Ausführungsbeispiel des der Erfindung zugrunde liegenden Gedankens lässt sich der Spacer beziehungsweise der Abstandhalter nach dem Öffnen der Umverpackung manuell aus der Öffnung zwischen der Dichtung und dem Magazin herausziehen, so dass die der Öffnung des Magazins zugeordnete Dichtung von ihrer inaktiven Position in ihre aktive, d.h. Dichtposition überführt wird. Gemäß dieser Ausführungsvariante erfolgt das Überführen der Dichtung von ihrem inaktiven Zustand im geschlossenen Zustand der Umverpackung in ihre aktive Position nach dem Öffnen der Umverpackung manuell durch den Benutzer.

Nach der Entnahme des Magazins aus der Umverpackung stellt der im Inneren des Magazins enthaltene Trockenmittelvorrat unter Berücksichtigung einer Restleckage und unter Berücksichtigung der Umgebungsbedingungen die Haltbarkeit der im Magazin enthaltenen Testelemente sicher.

Der mit der vorgeschlagenen Lösung einhergehende Vorteil ist darin zu erblicken, dass der im Magazin enthaltene Trockenmittelvorrat ab dem Zeitpunkt der Entnahme des Magazins aus der Umverpackung eine kleinere Wasserdampfbeladung, die herstellungsbedingt ist, aufweist. Dies hat zur Folge, dass bei Einsatz der erfindungsgemäß vorgeschlagenen Lösung entweder die Trockenmittelmenge im Magazin bei sonst identischen Bedingungen geringer ausgelegt werden kann, was eine Verkleinerung der Baugröße des die Testelemente aufnehmenden Magazins zur Folge hat, oder dass bei identischer in das Magazin eingebrachte Trockenmittelmenge die Haltbarkeit der darin aufgenommenen Testelemente erheblich gesteigert werden kann.

### Zeichnung

Anhand der Zeichnung wird die Erfindung nachstehend eingehender beschrieben.

Es zeigt:
- Figur 1: ein Umverpackungssystem gemäß des Standes der Technik im geschlossenen Zustand mit in der Umverpackung aufgenommenem Magazin für Testelemente,
- Figur 2: eine Detailansicht der Öffnung des Magazins des zwischen Magazingehäuse und Dichtung geführten Transportbandes,
- Figur 2.1: eine schematische Darstellung des Transportbandes mit in Seitenansicht nicht maßstabsgerecht wiedergegebenem Einzel-Testelement,
- Figur 3: die Darstellung des mit der Umverpackung verbundenen Spacers oder Abstandhalters zur Deaktivierung einer Dichtung am Gehäuse des Magazins bei verschlossener Umverpackung und
- Figur 4: die geöffnete Umverpackung mit aus der Öffnung entferntem Spacer oder Abstandhalter.

### Ausführungsbeispiele

Unter Magazin wird nachfolgend ein Aufnahmeelement verstanden, in welchem eine Anzahl von Testelementen aufgenommen ist. Das Magazin kann kassettenförmig ausgestaltet sein, wobei der Vorrat von Testelementen z. B. in Form von Wickelspulen mit einer Spule für unverbrauchte Testelemente und einer Spule für verbrauchte Testelemente ausgestattet ist. Das Magazin kann auch stapelförmig ausgebildet sein, wobei einzelne Testelemente, so z. B. in Streifenform, in vertikaler, horizontaler oder in relativ zueinander geneigter Anordnung aufgenommen sein können. Das Magazin kann auch als scheibenförmiger Körper beschaffen sein, an dessen Umfang einzelne Testelemente in Schlitzen aufgenommen sind, wobei ein Antrieb das scheibenförmig konfigurierte Magazin von Testelement zu Testelement weiterschaltet.

Unter Testelementen wird nachfolgend ein medizinisches Verbrauchsmaterial verstanden, welches z. B. in Streifenform im Magazin bevorratet ist und welches der Bestimmung eines Analyten in einer menschlichen Körperflüssigkeit, wie z. B. Blutzucker, Laktat oder Cholesterin und dergleichen, dient. Unter Öffnung wird nachfolgend eine Austrittsöffnung des z. B. in Teststreifenform vorliegenden medizinischen Verbrauchsmaterials aus dem Magazin verstanden, die z. B. als eine mittels einer an das Gehäuse des Magazins angestellten Dichtung oder als Längsschlitz mit Dichtlippen ausgebildet sein kann, um einen unerwünschten Feuchtigkeitseintritt in das Innere des die Testelemente aufnehmenden Magazins zu verhindern.

Unter Trockenmittelvorrat wird nachfolgend ein Trockenmittelsubstrat verstanden, welches der Aufnahme von Wasserdampf dient. Das Trockenmittel kann dabei in Pulverform vorliegen und in Beuteln, die in das Innere des Magazins oder in das Innere der Umverpackung eingelassen sind. Ferner kann das Trockenmittel als Trockenmittelkörper ausgebildet sein, welcher ein integraler Bestandteil des die Testelemente aufnehmenden Magazins ist.

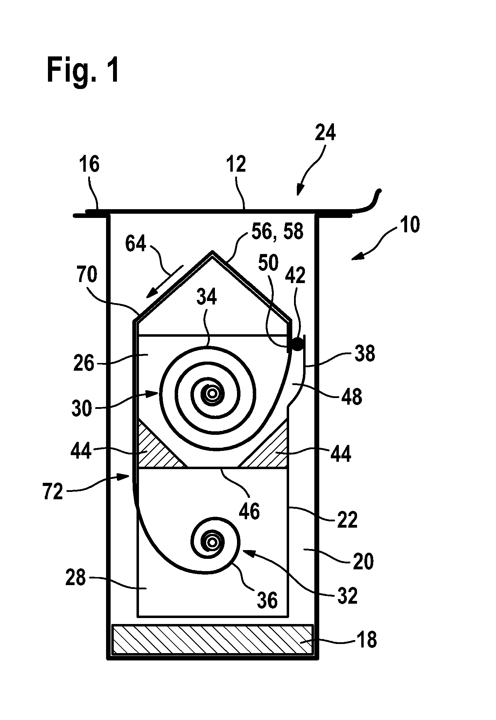

Figur 1 zeigt eine aus dem Stande der Technik bekannte Umverpackung zur Aufnahme eines Magazins mit Testelementen.

Eine Umverpackung 10 zur Aufnahme eines Magazins 22 wird in der Regel aus einem wasserdampfdichten Material hergestellt. Die Umverpackung 10 gemäß der Darstellung in Figur 1 umfasst einen hier schematisch dargestellten Verschluss 12, der an einer Seite 16 mit der Umverpackung 10 verbunden ist. Zur Entnahme des in der wasserdampfdichten Umverpackung 10 aufgenommenen Magazins 22 wird der im geschlossenen Zustand 24 dargestellte Verschluss 12 an einer Seite aufgetrennt. Die Umverpackung 10 weist einen ersten Trockenmittelvorrat 18 auf, der in der Darstellung gemäß Figur 1 auf dem Boden der Umverpackung 10 liegt. Der erste Trockenmittelvorrat 18 kann sowohl in Pulverform vorgesehen sein, wobei das Pulver in einen Beutel eingelassen sein kann. Daneben ist es auch möglich, den ersten Trockenmittelvorrat 18 als Trockenmittelkörper auszubilden, der in die Unterseite der Umverpackung 10 integriert ist.

In einem Hohlraum 20 der Umverpackung 10 befindet sich das Magazin 22. Das Magazin 22 weist einen ersten Magazinteil 26 und einen zweiten Magazinteil 28 auf. Im ersten Magazinteil 26 ist ein unverbrauchter Testelementvorrat 30 aufgenommen, während im zweiten Magazinteil 28 ein verbrauchter Testelementevorrat 32 aufgenommen ist. So kann z. B. bei einer kassettenförmigen Konfiguration des Magazins 22 der unverbrauchte Testelementvorrat 30 in Form eines Frischwickels 34 im ersten Magazinteil 26 aufgenommen sein, während im zweiten Magazinteil 28 der verbrauchte Testelementvorrat 32 in Form eines Gebrauchtwickels 36 aufgenommen ist. Eine Öffnung des Magazins 22 ist durch Bezugszeichen 48 gekennzeichnet. Die Öffnung wird mittels eines Federelementes 38 und einer mit dem Federelement 38 verbundenen Dichtung 42 verschlossen. In der Darstellung gemäß Figur 1 ist die Dichtung 42 an eine magazinseitige Auflage 50 des Magazins 22 angestellt. In der Darstellung gemäß Figur 1 werden die auf dem Frischwickel 34 des unverbrauchten Testelementvorrates 30 bevorrateten Einzeltestelemente mittels eines Transportbandes um die Außenseite des Gehäuses des Magazins 22 herumgeführt und passieren dabei die Öffnung 48. Nach erfolgter Benutzung des Testelementes durch den Anwender, d. h. nach Benetzen des Testelementes mit einer menschlichen Körperflüssigkeit, zur Bestimmung eines in dieser enthaltenen Analyten wird das verbrauchte Testelement auf dem Gebrauchtwickel 36 im zweiten Magazinteil 28 des Magazins 22 aufgewickelt.

Im ersten Magazinteil 26 des Magazins 22 ist ein zweiter Trockenmittelvorrat 44 aufgenommen. Der erste Magazinteil 26 und der zweite Magazinteil 28 sind durch eine Trennwand 46 voneinander getrennt. Der im ersten Magazinteil 26 bevorratete zweite Trockenmittelvorrat 44 dient der Entfeuchtung des unverbrauchten Testelementvorrates 30.

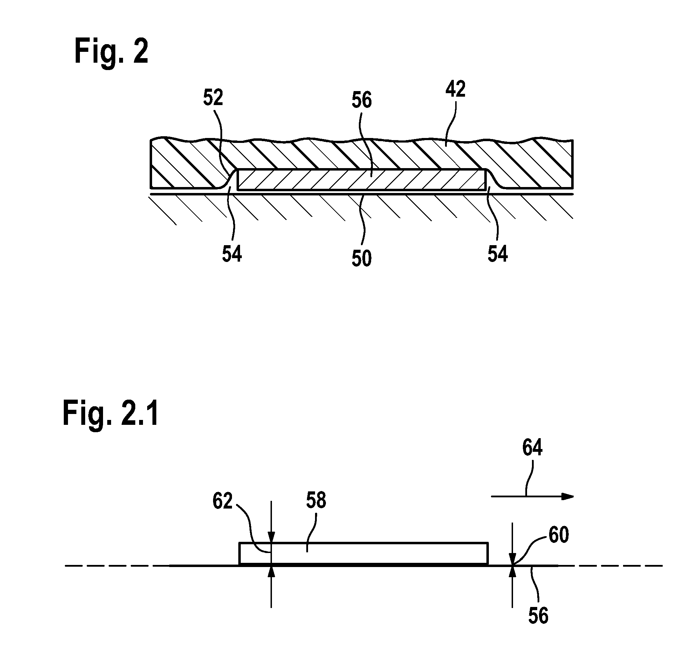

Der Darstellung gemäß Figur 2 ist eine in vergrößertem Maßstab wiedergegebene Darstellung der Öffnung des Magazins zu entnehmen.

Im in Figur 2 dargestellten Zustand ist die Dichtung 42, die durch das Federelement 38 beaufschlagt ist, an die magazinseitige Auflage 50 angestellt. Aufgrund der elastischen Eigenschaften des Dichtelementes 42 legt sich eine Kontaktseite 52 der Dichtung 42, wie in Figur 2 dargestellt, an ein Transportband 56 an. Damit wird die Öffnung 48 des Magazins 22 bis auf eine eine Restleckage ermöglichende minimale Größe reduziert. Die Restleckage, die durch die Öffnung 48 im aktiven Zustand 42 erfolgen kann, ist bedingt durch Zwickel 54, die sich neben einem Transportband 56 - um ein Beispiel eines Testelementträgers zu nennen - aufgrund der begrenzten Verformbarkeit der Dichtung 42 ergeben. Die Zwickel 54 ergeben sich in unterschiedlichen Geometrien abhängig vom durch das Federelement 38 erzeugten Anpressdruck und abhängig von der Alterung des elastischen Dichtungsmaterials der Dichtung 42. Mittels der durch die Blattfeder 38 beaufschlagten Dichtung 42 wird das Testelemente transportierende Transportband 56 an die magazinseitige Auflage 50 gedrückt.

Figur 2.1 zeigt in schematischer Darstellung eine Seitenansicht des in Figur 2 dargestellten Transportbandes.

Aus der Darstellung gemäß Figur 2.1 geht hervor, dass sich im Falle eines Transportbandes 56 als Träger eines einzelnen Testelementes 58 eine erste Dicke 60 - nämlich die Dicke des Transportbandes 56 - ergibt und die Kombination aus Testelement 58 und Transportband 56 eine zweite Dicke 62 aufweist. Daraus ergibt sich, dass die Öffnung 48 zwischen der Dichtung 42 und der magazinseitigen Auflage 50 des Magazins 22 alternierend auf die erste Dicke 60 und bei Passage eines Testelementes 58 auf die zweite Dicke 62 geöffnet wird.

Die in den Figuren 1 bis 2.1 beschriebene Ausführungsform eines Umverpackungssystems verhindert aufgrund der Anstellung der Dichtung 42 an die magazinseitige Auflage 50 einen Beitrag zur Trocknung des im Inneren des Magazins 22 aufgenommenen unverbrauchten Testelementvorrates 30, da die Öffnung 48 - wie in Figur 2 dargestellt - auf ein Mindestmaß reduziert ist. In diesem Falle ist zur Trocknung des unverbrauchten Testelementvorrates 30 im ersten Magazinteil 26 lediglich der zweite Trockenmittelvorrat 44 wirksam.

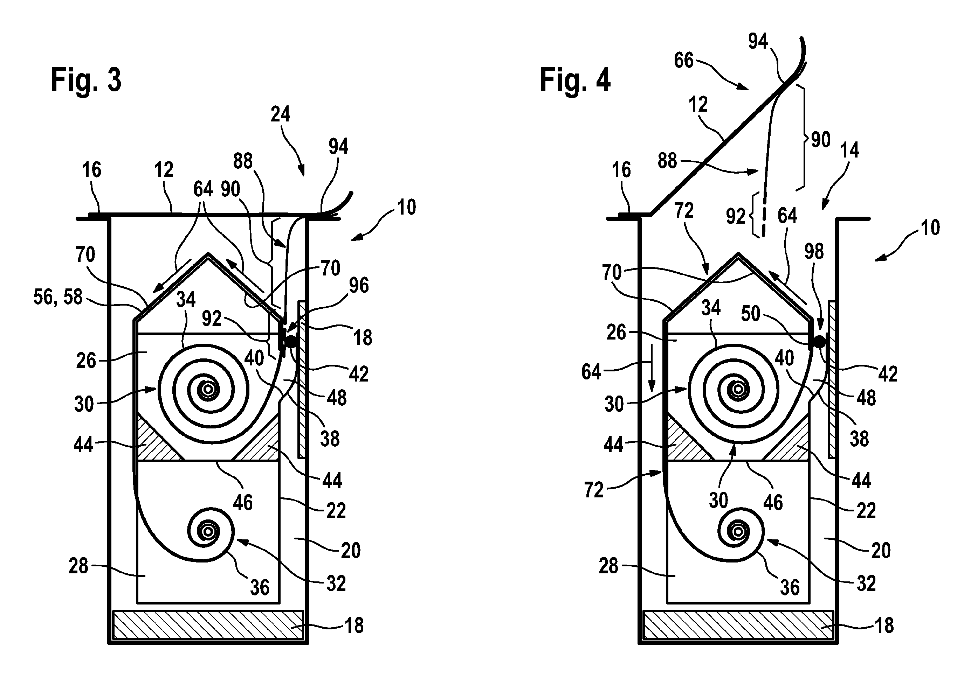

Figur 3 zeigt das Umverpackungssystem gemäß der vorliegenden Erfindung in verschlossenem Zustand.

Das Umverpackungssystem umfasst die Umverpackung 10, während Verschluss 12 sich in der Schließposition 24 befindet. Der Verschluss 12 ist an der Anlenkstelle 16 mit der wasserdampfdicht ausgeführten Umverpackung 10 verbunden. Im Hohlraum 20 der Umverpackung 10 sind sowohl der erste Trockenmittelvorrat 18 als auch das Magazin 22 aufgenommen. Das Magazin 22 ist durch die Trennwand 46 in den ersten Magazinteil 26 und den zweiten Magazinteil 28 unterteilt. Im ersten Magazinteil 26 ist der unverbrauchte Testelementvorrat 30 in Form des Frischwickels 34 aufgenommen, während sich im zweiten Magazinteil 28 der verbrauchte Testelementvorrat 32 in Form eines Gebrauchtwickels 36 befindet. Die Testelemente 58 sind in der Darstellung gemäß Figur 3 an einem auf dem Frischwickel 34 aufgespulten Transportband 56 ausgebildet, welches den ersten Magazinteil 26 durch die Öffnung 48 verlässt, um in Förderrichtung 64 um die Außenseite 70 des Magazins 22 in den zweiten Magazinteil 28 gefördert zu werden, in dem sich der verbrauchte Testelementvorrat 32 in Form des Gebrauchtwickels 36 befindet.

Aus der Darstellung gemäß Figur 3 ist entnehmbar, dass sich an der Unterseite des Verschlusses 12 an einer Verbindungsstelle 94 ein Spacer/Abstandhalter 88 befindet. An der Verbindungsstelle 94 ist dieser mit der Unterseite des Verschlusses 12 verbunden. Das der Verbindungsstelle 94 gegenüberliegende Ende des Spacers/Abstandhalters 88 erstreckt sich in die Öffnung 48 des Magazins 22. Der Abstandhalter 88 kann einen ersten Abschnitt 90 umfassen sowie einen zweiten Abschnitt 92, der bevorzugt aus einem porösen, eine Wasserdampfdiffusion ermöglichenden Material gefertigt wird. Der zweite Abschnitt 92 des Spacers/Abstandhalters 88 kann in Form eines porösen Bandes, eines Vlieses, einer Fadenstruktur oder dergleichen ausgeführt werden. Am zweiten Abschnitt 92 des Spacers/Abstandhalters 88 kann auch eine rampenförmig ausgebildete Erhebung, ein Keil, ein Zylinder, ein Klotz oder drgl. ausgebildet werden, ferner kann der zweite Abschnitt 92 des Spacers/Abstandhalters 88 auch in Form eines Bandes mit einer Durchbrechung so zum Beispiel einem Loch, einem Langloch oder einer Anzahl von Schlitzen ausgebildet sein. Der Spacer/Abstandhalter 88 oder dessen zweiter Abschnitt 92 wird bevorzugt aus einem chemisch inaktiven Material gefertigt. Dieses Material kann aufgrund seiner Materialeigenschaften eine Wasserdampfdiffusion ermöglichen, andererseits kann durch die vorstehend aufgezählten Varianten hinsichtlich der Geometrie des Spacer/Abstandhalters 88 bzw. von dessen zweiten Abschnitt 92 in Gestalt eines Bandes mit einer Öffnung, seien es Löcher, Langlöcher, Schlitz oder drgl. oder durch Versehen mit einem Keil, einer Rampe, einem Zylinder oder drgl. eine Wasserdampfdiffusion aus dem Inneren des Magazins 22 in die verschlossene Umverpackung 10 ermöglicht werden.

Aufgrund des sich in die Öffnung 48 des Magazins 22 erstreckenden zweiten Abschnitts 92 des Spacers/Abstandhalters 88 nimmt die Dichtungsstelle 42 einen deaktivierten Zustand 96 ein. Dies bedeutet, dass die Öffnung 48 im Bereich der Dichtung 42 und der Außenseite 70 des Magazins 22 aufgrund des in diese eingeführten Spacers/Abstandhalters 88 geöffnet steht. Im deaktivierten Zustand 96 der Dichtung 42 besteht demnach eine Verbindung des im ersten Magazinteil 26 aufgenommenen zweiten Trockenmittelvorrats 44 und dem ersten Trockenmittelvorrat 18, der sich im Hohlraum 20 der wasserdampfdicht ausgeführten Umverpackung 10 befindet. Dadurch wird erreicht, dass der im ersten Magazinteil 26 bevorratete und verbrauchte Testelementvorrat 30 nicht nur durch den zweiten Trockenmittelvorrat 44 innerhalb des Magazins 22, sondern auch durch den in der Umverpackung 10 vorgesehenen ersten Trockenmittelvorrat 18 entfeuchtet werden kann. Solange der Verschluss 12 der Umverpackung 10 in der Schließposition 24 bleibt, ragt ein Ende des Spacers/Abstandhalters 88 in die Öffnung 48 und vergrößert diese derart, dass der erste Trockenmittelvorrat 18 im Hohlraum 20 der Umverpackung 10 bei der Entfeuchtung des unverbrauchten Testelementvorrats 30 des Magazins 22 mitwirkt. Damit kann erreicht werden, dass im verschlossenen Zustand des Umverpackungssystems gemäß Figur 3 der zweite Trockenmittelvorrat 44 innerhalb des ersten Magazinteils 26 des Magazins 22 eine kleinere, herstellungsbedingte Vorbelastung hinsichtlich einer Feuchtigkeitsbeladung aufweist, wenn die Umverpackung 10 geöffnet wird und somit der erste Trockenmittelvorrat 18 nicht mehr wirksam ist. Dadurch kann entweder die Menge des im ersten Magazinteil 26 bevorrateten zweiten Trockenmittelvorrates 44 reduziert werden bei sonst identischen Bedingungen, so dass das Magazin 22 insgesamt gesehen kleiner ausgelegt werden kann oder bei identischer Menge des zweiten Trockenmittelvorrates 44 die Haltbarkeit des unverbrauchten Testelementvorrates 30 erhöht werden, da die Vorbelastung des zweiten Trockenmittelvorrates 44 geringer geworden ist.

Der Darstellung gemäß Figur 4 ist das Umverpackungssystem gemäß der Darstellung in Figur 3 in geöffnetem Zustand zu entnehmen.

In Figur 4 ist der Verschluss 12 der wasserdampfdicht ausgeführten Umverpackung 10 geöffnet, angedeutet durch Bezugszeichen 66. Während des Öffnungsvorganges wird der Spacer/Abstandhalter 88, der an der Verbindungsstelle 94 mit dem Verschluss 12 verbunden ist, aus der Öffnung 48, insbesondere aus dem Bereich zwischen der Dichtung 42 und der magazinseitigen Auflage 50, herausgezogen. Die Dichtung 42 nimmt nunmehr ihren aktivierten Zustand 98 ein und dichtet den ersten Teil 26 des Magazins 22 ab, in dem der unverbrauchte Testelementvorrat 30 enthalten ist. Der Spacer/Abstandhalter 88 nimmt seine in Figur 4 dargestellte Position ein. Da sich der zweite Abschnitt 92 des Spacers/Abstandhalters 88 nicht mehr zwischen der Dichtung 42 und der magazinseitigen Auflage 50 befindet, nimmt die Öffnung 48 zwischen der Dichtung 42 und der magazinseitigen Auflage 50 wieder ihre ursprüngliche Größe an, bedingt durch die Vorspannkraft des als Blattfeder 40 ausführbaren Federelementes 38. Andererseits kann der Spacer/Abstandhalter 88 in einer weiteren Ausführungsvariante des der Erfindung zugrunde liegenden Gedankens nicht mit dem Verschluss 12 verbunden sein. In diesem Falle lässt sich der Spacer/Abstandhalter 88 vom Benutzer nach Öffnen der Umverpackung 10 manuell aus der Öffnung 48 am Magazin 22 herausziehen. Durch diesen manuellen Eingriff des Benutzers erfolgt ebenfalls eine Überführung der Dichtung 42 von ihrem deaktivierten Zustand 96 in ihren aktivierten Zustand 48. Nach der Entnahme des Magazins 22 aus der geöffneten Umverpackung 10 stellt die Dichtung 42 sicher, dass der im Magazin 22 enthaltene Vorrat von Testelementen 58 gegen die Umgebung abgedichtet ist. Im geöffneten Zustand der wasserdampfdicht ausgeführten Umverpackung 10 wird das Magazin 22 mit darin aufgenommenem unverbrauchten Testelementvorrat 30 aus der Umverpackung 10 entnommen. Die Entfeuchtung des unverbrauchten Testelementvorrates 30 erfolgt nach dem Öffnen der wasserdampfdichten Umverpackung 10 nunmehr durch den zweiten, im ersten Magazinteil 26 aufgenommenen Trockenmittelvorrat 44, der in diesem in Form eines Trockenmittelkörpers 82 oder auch in Beutel eingefüllter Pulverform vorliegen kann.

Aufgrund der im geschlossenen Zustand 24 durch den inaktiven Zustand 96 der Dichtung 42 erfolgenden Entfeuchtung des unverbrauchten Testelementvorrates 30 durch den ersten Trockenmittelvorrat 18 und den zweiten Trockenmittelvorrat 44 weist der zweite Trockenmittelvorrat 44 nach Öffnen der Umverpackung 10 gemäß der Darstellung in Figur 4 eine kleinere Vorbelastung auf, wodurch die Haltbarkeit (On-Board-Stability) des im ersten Magazinteils 26 des Magazins 22 aufgenommenen unverbrauchten Testelementvorrates 30 erhöht werden kann. Aufgrund der geringeren Vorbelastung des zweiten Trockenmittelvorrates 44 im Magazin 22 kann nun einerseits die Menge des in das Magazin 22 einzubringenden zweiten Trockenmittelvorrates 44 verringert werden, wodurch sich dessen Baugröße verkleinern lässt. Andererseits ist es durch die erfindungsgemäß vorgeschlagene Zusammenschaltung der beiden Trockenmittelvorräte 18 und 44 im geschlossenen Zustand 24 des Umverpackungssystems möglich, die Haltbarkeit des unverbrauchten Testelementvorrates 30 nach Öffnen des Verschlusses 12 der wasserdampfdichten Umverpackung 10 zu erhöhen. Dies wiederum ist einerseits anwenderfreundlich und gestattet andererseits auch eine Erhöhung der Anzahl der im Magazin 22 zu bevorratenden Testelemente 58, sei es in Spulenform, wie vorstehend dargestellt, sei es in Form gestapelter Testelemente 58 in vertikaler, horizontaler oder geneigter Anordnung innerhalb eines Magazins 22.

### Bezugszeichenliste

- 10: Umverpackung
- 12: Verschluss
- 14: Entnahmeöffnung
- 16: Anlenkstelle Verschluss
- 18: erster Trockenmittelvorrat
- 20: Hohlraum Umverpackung 10
- 22: Magazin
- 24: Schließposition Verschluss 12
- 26: erster Magazinteil
- 28: zweiter Magazinteil
- 30: unverbrauchter
Testelementvorrat
- 32: verbrauchter
Testelementvorrat
- 34: Frischwickel
- 36: Gebrauchtwickel
- 38: Federelement
- 40: Blattfeder
- 42: Dichtung
- 44: zweiter Trockenmittelvorrat
- 46: Trennwand
- 48: Öffnung
- 50: magazinseitige Auflage
- 52: Kontaktseite Dichtung
- 54: Zwickel
- 56: Transportband
- 58: Testelement
- 60: Dicke Transportband
- 62: Dicke Transportband +
Testelement
- 64: Förderrichtung
- 66: Offenstellung
- 70: Außenseite Magazin
- 72: Transportbandpfad
- 88: Spacer/Abstandhalter
- 90: erster Abschnitt Spacer

- 92: zweiter Abschnitt Spacer
- 94: Verbindungsstelle Verschluss 12
- 96: deaktivierter Zustand
Dichtung 42
- 98: aktivierter Zustand Dichtung 42

## Patentansprüche

1. Umverpackungssystem für medizinisches Verbrauchsmaterial, insbesondere Testelemente (58), mit einer Umverpackung (10), die einen ersten Trockenmittelvorrat (18) aufnimmt, und einem Magazin (22) mit einem unverbrauchten Testelementvorrat (30), **dadurch gekennzeichnet, dass** im geschlossenen Zustand (24) der Umverpackung (10) der erste Trockenmittelvorrat (18) der Umverpackung (10) auf das Innere des Magazins (22) über eine eine Wasserdampfdiffusion ermöglichende Verbindung (96) einwirkt.

2. Umverpackungssystem gemäß Anspruch 1, **dadurch gekennzeichnet, dass** das Magazin (22) einen zweiten Trockenmittelvorrat (44) enthält.

3. Umverpackungssystem gemäß Anspruch 1, **dadurch gekennzeichnet, dass** das Magazin (22) eine Dichtung (42) aufweist, die im geschlossenen Zustand (24) der Umverpackung (10) einen eine Wasserdampfdiffusion in die Umverpackung (10) ermöglichenden, deaktivierten Zustand (96) annimmt.

4. Umverpackungssystem gemäß Anspruch 3, **dadurch gekennzeichnet, dass** die Dichtung (42) durch ein Federelement (38) beaufschlagt ist.

5. Umverpackungssystem gemäß der Ansprüche 3 oder 4, **dadurch gekennzeichnet, dass** die Dichtung (42) in eine Ausnehmung (76) des Magazins (22) eingelassen oder am Federelement (38) befestigt ist.

6. Umverpackungssystem gemäß Anspruch 3, **dadurch gekennzeichnet, dass** an der Umverpackung (10) ein Spacer/Abstandhalter (88) ausgebildet ist, der im geschlossenen Zustand (24) der Umverpackung (10) die Dichtung (42) in ihrem deaktivierten Zustand (96) hält.

7. Umverpackungssystem gemäß Anspruch 6, **dadurch gekennzeichnet, dass** der Spacer/Abstandhalter (88) einen ersten Abschnitt (90) und einen zweiten Abschnitt (92) aufweist, wobei der zweite Abschnitt (92) aus einem eine Wasserdampfdiffusion ermöglichenden Material gefertigt ist oder dessen Geometrie eine Wasserdampfdiffuision ermöglicht.

8. Umverpackungssystem gemäß Anspruch 7, **dadurch gekennzeichnet, dass** der zweite Abschnitt (92) als poröses Band, Vlies, Fadenstruktur oder Gewebe ausgebildet ist

9. Umverpackungssystem gemäß Anspruch 6, **dadurch gekennzeichnet, dass** der erste Abschnitt (90) des Spacers/Abstandhalters (88) an einer Verbindungsstelle (94) an dem zu öffnenden Teil (12) der Umverpackung (10) befestigt ist.

10. Umverpackungssystem gemäß Anspruch 1, **dadurch gekennzeichnet, dass** der erste und der zweite Trockenmittelvorrat (18, 44) als Trockenmittelkörper in der Umverpackung (10) und im Magazin (22) ausgeführt sind.

11. Umverpackungssystem gemäß Anspruch 1, **dadurch gekennzeichnet, dass** die Umverpackung (10) aus einem wasserdampfdichten Material gefertigt ist.

12. Umverpackungssystem gemäß Anspruch 3, **dadurch gekennzeichnet, dass** die Dichtung (42) im deaktivierten Zustand (96) einen vergrößerten Querschnitt einer Öffnung (48) zum unverbrauchten Testelementvorrat (30) im Magazin (22) freihält.

13. Umverpackungssystem gemäß Anspruch 3, **dadurch gekennzeichnet, dass** die Dichtung (42) beim Öffnen der Umverpackung (10) in einen aktivierten Zustand (98) überführt wird.

14. Umverpackungssystem gemäß Anspruch 13, **dadurch gekennzeichnet, dass** die Dichtung (42) im aktivierten Zustand (98) an einen Teil des in der Öffnung (48) befindlichen unverbrauchten Testelementvorrats (30) angestellt ist.

15. Umverpackungssystem gemäß Anspruch 1, **dadurch gekennzeichnet, dass** der unverbrauchte Testelementvorrat (30) in Gestalt eines Frischwickels (34) eines Transportbandes (56) mit Testelementen (58) in einem kassettenartigen Magazin (22) aufgenommen ist.

16. Umverpackungssystem gemäß Anspruch 1, **dadurch gekennzeichnet, dass** der erste Trockenmittelvorrat (18) im Hohlraum (20) der Umverpackung (10) einer Öffnung (48) des Magazins (22) gegenüberliegend angeordnet ist.

17. Umverpackungssystem gemäß Anspruch 1, **dadurch gekennzeichnet, dass** der zweite Trockenmittelvorrat (44) zwischen einem ersten Teil (26) des Magazins (22) enthalten ist, der von einem zweiten Teil (28) des Magazins (22) getrennt ist.

18. Verwendung des Umverpackungssystems gemäß einem der Ansprüche 1 bis 17 zur Aufnahme eines unverbrauchten Testelementvorrates (30), insbesondere Testelemente (58) zur Bestimmung von Blutzucker, Cholesterin, Laktatwerten, Bilirubin, Triglyceride, Amylase, Harnsäure, Harnstoff, Kalium, HDL-Cholesterin oder Creatin-Kinase.

19. Verfahren zur Verlängerung der Haltbarkeit von medizinischem Verbrauchsmaterial, insbesondere von Testelementen (58), die in einer Umverpackung (10) aufgenommen sind, mit nachfolgenden Verfahrensschritten:
a) im geschlossenen Zustand (24) der Umverpackung (10) wird eine eine Wasserdampfdiffusion ermöglichende Verbindung (96) zwischen einem in der Umverpackung (10) enthaltenen ersten Trockenmittelvorrat (18) und dem Inneren eines die Testelemente (58) aufnehmenden Magazins (22) geschaffen,
b) durch das Öffnen der Umverpackung (10) oder nach dem Öffnen der Umverpackung (10) wird die eine Wasserdampfdiffusion ermöglichende Verbindung (96) geschlossen.

20. Verfahren gemäß Anspruch 19, **dadurch gekennzeichnet, dass** gemäß Verfahrensschritt (b) die eine Wasserdampfdiffusion ermöglichende Verbindung (96) durch das Öffnen (66) eines Verschlusses (12) geschlossen wird.

21. Verfahren gemäß Anspruch 19, **dadurch gekennzeichnet, dass** gemäß Verfahrensschritt (b) die eine Wasserdampfdiffusion ermöglichende Verbindung (96) manuell geschlossen wird.
